# EUROPEAN PATENT APPLICATION

(11) **EP 4 253 522 A1**
(43) Date of publication of application: **04.10.2023**
(21) Application number: 22305432.1
(22) Date of filing: 01.04.2022
(51) Int. Cl.: C12M 1/42

(54) **ACOUSTIC METHOD FOR TRANSDUCTION AND TRANSFECTION**

(71) Applicant: Aenitis Technologies, 77290 Mitry-Mory (FR)
(72) Inventor: VINCENT, Emmanuel, 77290 Mitry-Mory (FR); BERTIN, Nicolas, 77290 Mitry-Mory (FR); GORJI, Azita, 77290 Mitry-Mory (FR)
(74) Representative: Icosa

(57) **Abstract**

The present invention relates to a method for introducing foreign nucleic acids (3) into cells (2) assisted with acoustophoresis and comprising a step of sweeping acoustic waves frequency from a first frequency f₁ to a second frequency f₂ using a sweep time ranging from 1 ms to 100 ms, f₂ being superior to f₁. The present invention also relates to a method for performing transduction of cells (2), cells obtained by the method of the invention and an acoustophoresis device (1) for introducing foreign nucleic acids (3) into cells (2) comprising a chamber (11), at least two inlets (121, 122), at least two outlets (131, 132) and at least one acoustic wave generator (14) is configured to sweep the acoustic waves frequency from a first frequency f₁ to a second frequency f₂ using a sweep time ranging from 1 ms to 100 ms, f₂ being superior to f₁.

## Description

### FIELD OF INVENTION

The present invention relates to methods and devices for introducing foreign nucleic acids into cells using acoustophoresis. In particular, the present invention relates to methods and devices for method for transduction or transfection of cells using acoustophoresis. The present invention also relates to modified cells obtained by said methods.

### BACKGROUND OF INVENTION

Transfection and transduction of cells constitute an essential tool for the understanding of cell biology and therapeutic modulation of gene expression. Quite a few techniques, classified as viral (transduction) and nonviral (transfection), have been developed to introduce nucleic acids into target cells. The transduction approach is a very effective method for gene delivery; however, it is plagued by immune response and insertional mutagenesis. Because of the risky concerns of using viral vectors, the transfection approach has gradually gained attention, although the achievable transfection efficiency is not as great as transduction, while transduction is a lot more expensive than transfection.

Viral vectors such as retroviruses and adenoviruses have been shown to be efficient in transduction. However, these viral vectors are hard to provide, thus expensive and conventional transduction methods exhibit a high multiplicity of infection, *i.e.* the viral vectors are used in large excesses compared to cells.

Among reported nonviral techniques such as electroporation, microinjection, lipofection, etc. An acoustically induced transfection method, known as sonoporation, has been developed, wherein acoustic cavitation of microbubbles is used to enhance delivery of nucleic acids. Such ultrasound-mediated transfection, substantially relying on acoustic cavitation has severe drawbacks as the cell membrane may be seriously disrupted and subsequently cause lethal damage. Thus, in conventional acoustically induced transfection, the viability of the cells is quite low.

Furthermore, current transduction and transfection methods are not readily scalable for the large numbers of cells required in clinical protocols, have relatively high cost and low gene transfer efficiency, and poor ability to be scaled up for commercialization.

There is thus a need for gene transfer method, either by transfection or by transduction, having one or more of the following advantages: exhibiting high gene transfer efficiency, reduced cost, low multiplicity of infection, high cell viability, being readily scalable by allowing treatment of a large number of cells in a reduced time.

The Applicant has found that this need could be met with a non-invasive, acoustophoresis assisted method for introducing foreign nucleic acids into cells comprising forced confinement of cells and foreign nucleic acids in a defined volume by deflection of said cells to a suspension comprising said nucleic acids.

### SUMMARY

The present invention relates to a method for introducing foreign nucleic acids into cells comprising:
i. providing an acoustophoresis device comprising:
   - a chamber configured to be associated with at least one acoustic wave generator for generating acoustic waves within the chamber;
   - at least one acoustic wave generator configured to generate acoustic waves within the chamber;
   - at least two inlets in fluid communication with the chamber, said inlets comprising a first inlet and at least one second inlet;
   - at least two outlets in fluid communication with the chamber, said outlets comprising a first outlet and at least one second outlet;
ii. injecting a suspension comprising foreign nucleic acids in the chamber at the first inlet and injecting a cell suspension in the chamber at the at least one second inlet;
iii. applying an acoustic field by generating acoustic waves inside the chamber;
iv. sweeping the acoustic waves frequency from a first frequency f₁ to a second frequency f₂ using a sweep time ranging from 1 ms to 100 ms, f₂ being superior to f₁;
v. collecting a suspension comprising cells modified by foreign nucleic acids at the first outlet.

In one embodiment, the nucleic acids are carried in a viral vector, or a macrovesicle. In one embodiment, first frequency f₁ and second frequency f₂ are each ranging from 0.1 MHz to 4 MHz. In one embodiment, said method further comprises an additional step after step (iv) of sweeping the acoustic waves frequency from the second frequency f₂ to a third frequency f₃ using a sweep time ranging from 0.5 ms to 10 ms, f₂ being superior to f₃. In one embodiment, first frequency f₁ is equal to third frequency f₃. In one embodiment, power applied to the acoustic wave generator is ranging from 0.2 W to 50 W. In one embodiment, the acoustic waves generated are volumetric acoustic waves, surface acoustic waves, standing surface acoustic waves, multi-dimensional acoustic waves or standing acoustic waves. In one embodiment, multiplicity of infection is ranging from 0.5 to 30. In one embodiment, the cells are selected among Chinese hamster ovary (CHO) cells, NSO hybridoma cells, baby hamster kidney (BHK) cells, human cells, regulatory T- cells, helper T-cells, cytotoxic T-cells, memory T-cells, effector T-cells, gamma delta T- cells, Jurkat T-cells, CAR-T cells, B cells, or NK cells, peripheral blood mononuclear cells (PBMCs), HEK293T, algae, plant cells, Tumor Infiltrating Lymphocytes (TIL), or bacteria.

The present invention also relates to a method for performing transduction of cells, comprising:
i. providing an acoustophoresis device comprising:
   - a chamber configured to be associated with at least one acoustic wave generator for generating acoustic waves within the chamber;
   - at least one acoustic wave generator configured to generate acoustic waves within the chamber;
   - at least two inlets in fluid communication with the chamber, said inlets comprising a first inlet and at least one second inlet;
   - at least two outlets in fluid communication with the chamber, said outlets comprising a first outlet and at least one second outlet;
ii. injecting a suspension of viral vectors comprising nucleic acids in the chamber at the first inlet and injecting a cell suspension in the chamber at the at least one second inlet;
iii. applying an acoustic field by generating acoustic waves inside the chamber;
iv. sweeping the acoustic waves frequency from a first frequency f₁ to a second frequency f₂ using a sweep time ranging from 1 ms to 100 ms, f₂ being superior to f₁;
v. collecting a suspension comprising cells modified by nucleic acids at the first outlet.

In one embodiment, first frequency f₁ and second frequency f₂ are each ranging from 0.1 MHz to 4 MHz. In one embodiment, said method further comprises an additional step after step (iv) of sweeping the acoustic waves frequency from the second frequency f2 to a third frequency f₃ using a sweep time ranging from 0.5 ms to 10 ms, f₂ being superior to f₃. In one embodiment, the cells are selected among Chinese hamster ovary (CHO) cells, NSO hybridoma cells, baby hamster kidney (BHK) cells, human cells, regulatory T- cells, helper T-cells, cytotoxic T-cells, memory T-cells, effector T-cells, gamma delta T- cells, Jurkat T-cells, CAR-T cells, B cells, or NK cells, peripheral blood mononuclear cells (PBMCs), algae, plant cells, HEK293T, Tumor Infiltrating Lymphocytes (TIL), or bacteria.

The present invention also relates to cells obtained by the method according to the invention.

The present invention also relates to an acoustophoresis device for introducing foreign nucleic acids into cells comprising:
- a chamber configured to be associated with at least one acoustic wave generator for generating acoustic waves within the chamber, said chamber extending along a longitudinal axis;
- at least one acoustic wave generator configured to generate acoustic waves within the chamber;
- at least two inlets in fluid communication with the chamber, said inlets comprising a first inlet for introducing a suspension comprising foreign nucleic acids in the chamber, and at least one second inlet for introducing a cell suspension in the chamber;
- at least two outlets in fluid communication with the chamber, said outlets comprising a first outlet for collecting a suspension comprising cells modified by foreign nucleic acids and at least one second outlet;
wherein the at least one acoustic wave generator is configured to sweep the acoustic waves frequency from a first frequency f₁ to a second frequency f₂ using a sweep time ranging from 1 ms to 100 ms, f₂ being superior to f₁.

### DEFINITIONS

In the present invention, the following terms have the following meanings:

**"Longitudinal axis of the chamber"** refers to the line joining the set of center of the cross sections of the chamber. The longitudinal axis of the chamber may be straight or curved and may be contained in a plane which may be a plane of symmetry for some or all of the cross sections of the chamber.

**"Multiplicity of infection"** (MOI) refers to the ratio of agents (e.g. phage or more generally virus, bacteria) to infection targets (e.g. cell). In the present invention, the multiplicity of infection refers to the number of viral vector particles per cell present in the chamber of the acoustophoresis device.

"**Naked nucleic acids**" refers to free nucleic acids (herein also called plasmids), *i.e.* nucleic acids that are not contained or carried in a viral vector or a microvesicle.

"**Nucleic acids**" refers to DNA or RNA.

"**Transduction**" refers to a reaction consisting of the transfer of genetic material (e.g. nucleic acids such as DNA or RNA) from a donor to a recipient (e.g. cell) via a viral vector.

"**Transfection**": refers to a reaction consisting of the introduction of nucleic acids (e.g. DNA or RNA) into eukaryotic cells.

### DETAILED DESCRIPTION

This invention relates to a method for introducing foreign nucleic acids into cells.

Said method comprises:
i. providing an acoustophoresis device comprising:
   - a chamber configured to be associated with at least one acoustic wave generator for generating acoustic waves within the chamber;
   - at least one acoustic wave generator configured to generate acoustic waves within the chamber;
   - at least two inlets in fluid communication with the chamber, said inlets comprising a first inlet and at least one second inlet;
   - at least two outlets in fluid communication with the chamber, said outlets comprising a first outlet and at least one second outlet;
ii. injecting a suspension comprising foreign nucleic acids in the chamber at the first inlet and injecting a cell suspension in the chamber at the at least one second inlet;
iii. applying an acoustic field by generating acoustic waves inside the chamber;
iv. sweeping the acoustic waves frequency from a first frequency f₁ to a second frequency f₂ using a sweep time ranging from 1 ms to 100 ms, f₂ being superior to f₁;
v. collecting a suspension comprising cells modified by foreign nucleic acids at the first outlet.

The method of the invention uses an acoustophoresis device (as described hereafter) comprising an acoustic wave generator (or more) to perform transfection or transduction of cells. The acoustophoretic device is used to bring cells together with foreign nucleic acids (either DNA or RNA, or both) in closed proximity, i.e. by confining cells and foreign nucleic acids in a same layer of fluid. This increases the probability of contact between cells and foreign nucleic acids, thus allowing the nucleic acids to be easily transferred into the cells. This confinement of cells with foreign nucleic acids is advantageously performed using a non-invasive and pressure less method to boost transduction or transfection.

The suspension comprising foreign nucleic acids and the suspension comprising cells are injected at distinct inlets and flow through the chamber according to a laminar flow, resulting in at least two layers of fluid flowing through said chamber without mixing.

The chamber extends along a longitudinal axis and comprises two opposing walls extending along said longitudinal axis (herein called longitudinal walls) and two opposing walls being perpendicular to said longitudinal axis (herein called transversal walls). The longitudinal walls comprise the transfer wall, *i.e.* the wall of the chamber coupled to the acoustic wave generator, and the opposite wall. The first inlet is preferably located at equidistance from those two longitudinal walls so that the suspension comprising foreign nucleic acids being injected at said first inlet forms a central layer of fluid in the chamber.

Acoustophoresis is used for the manipulation of particles in a fluid using acoustic waves. The application of an acoustic field inside the chamber, by generating acoustic waves using the acoustic wave generator, induces localized regions of high and low pressure are created inside the chamber and, in particular, an acoustic pressure node is created in the center of the chamber (depending on the chosen frequency at which the acoustic wave generator operates), a focusing plane is located on the acoustic pressure node, and acoustic radiation forces (ARF) are generated in the chamber. The force exerted on the particles also depends on their size, with larger particles experiencing larger forces. The magnitude of the force depends on the particle density and compressibility relative to the fluid medium, and increases with the particle volume. This results in a movement of the particles present in said chamber depending on their size.

In the method of the invention, the cells, having a micrometric size, experience far larger ARF than the foreign nucleic acids, having a nanometric size. Thus, only the cells are moved by the acoustic field application due to their micrometric size, whereas the foreign nucleic acids are not affected by the acoustic field application due to their smaller, nanometric, size. This allows for the deflection only of the cells within the chamber. The cells are being confined in a small fluid volume where the nucleic acids are already present, with no contact or pressure, with a continuous flow to transduce or transfect them. Advantageously, this avoids dilution of the foreign nucleic acids in larger fluid volumes, which would reduce the chances of nucleic acids/cell encounters.

Upon application of the acoustic field within the chamber, the cells are deflected from their initial suspension to the central layer of fluid, *i.e.* to the suspension comprising foreign nucleic acids, as the ARF push the cells towards the central pressure node with a force of up to a hundred times gravity equivalent, typically a force larger than the combined effect of fluid drag force and gravitational force. This forced confinement of cells and foreign nucleic acids in the same layer of fluid greatly encourages transfer of said foreign nucleic acids to the cells, resulting in modified cells.

The suspension comprising cells thus modified by foreign nucleic acids then flows towards the first outlet, while the suspension depleted in cells flows towards the at least one second outlet to be collected.

The invention relies on the voluntary deflection of cells to a suspension comprising foreign nucleic acids in a continuous flow of suspensions. This is particularly advantageous as it allows a better transfer of said foreign nucleic acids to the cells, for example an enhanced transduction efficiency when said foreign nucleic acids are comprised in viral vectors, or an enhanced transfection efficiency in the case of naked foreign nucleic acids.

In the method of the invention, the cells and the foreign nucleic acids both flow through the chamber according to a continuous fluid flow (at the same or different flow rates), so that the method of the invention does not rely on gravitational settling to collect modified cells, *i.e.* cells do not form clusters that fall out of the acoustic field to the bottom of the chamber. Advantageously, the same chamber can be used to treat small or big volumes of cells.

Sweeping the acoustic waves frequency from a first frequency f₁ to a second frequency f₂ avoids parasitic fluid flows inside the chamber which are detrimental to the concentration of the cells in the center of the chamber. In particular, this sweeping step avoids acoustic streaming inside the chamber, which refers to the fluid flow that results within the acoustic chamber when the fluid absorbs the acoustic energy that is transmitted by the acoustic wave generator. When acoustic streaming is generated, it results in circulatory motion or vortices that can cause stirring in the fluid mixture. This phenomenon is nonlinear, and disturbs the laminar flow inside the chamber. In the method of the invention, the frequencies are preferably around 1 MHz, and acoustic streaming appears essentially at frequencies under 1 MHz. Circulatory motions in the chamber resulting from acoustic streaming would in priority disturb the smallest biological objects, i.e. the foreign nucleic acids, disrupting the laminar flow of the fluid layers and diluting the foreign nucleic acids in the chamber. This would result in poor proximity between cells and foreign nucleic acids. Frequency sweeping is then essential to avoid the appearance of this phenomenon.

In an advantageous manner, the method of the invention has a limited number of steps and is easy to implement. It also exhibits reduce cost, increase transduction or transfection efficiency, high cell viability and low MOI.

In other words, the method of the invention is a method for boosting the introduction of foreign nucleic acids into cells.

Measuring protein expression confirms that the foreign nucleic acids were successfully introduced into cells, for example by Western blot or immunostaining.

According to one embodiment, in step (iv), the acoustic waves frequency is continuously swept from a first frequency f₁ to a second frequency f₂ using a sweep time ranging from 1 ms to 100 ms, *i.e.* all the frequencies in the range [f₁-f₂] are swept.

According to one embodiment, first frequency f₁ and second frequency f₂ are each ranging from 0.1 MHz to 4 MHz, preferably from 0.5 MHz to 2 MHz, more preferably from 0.8 MHz to 1.5 MHz, even more preferably from 0.9 MHz to 1.1 MHz.

Low frequencies, e.g. around 1 MHz, are particularly suitable for large cells. As the thickness of the channel is inversely proportional to the frequency, a low frequency allows for thicker channels and therefore higher throughputs. For example, for cells having a diameter higher than 20 µm, frequencies lower than or equal to 1 MHz shows a good deflection efficiency.

In a preferred configuration of this embodiment, first frequency f₁ is ranging from 0.5 MHz to 1.5 MHz, more preferably from 0.8 MHz to 1.2 MHz, even more preferably from 0.9 MHz to 1 MHz.

In another preferred configuration of this embodiment, second frequency f₂ is ranging from 0.9 MHz to 2 MHz, more preferably from 1 MHz to 1.5 MHz, even more preferably from 1.05 MHz to 1.2 MHz.

In another preferred configuration of this embodiment, the range [fi-f2] is centered at the resonance frequency fᵣₑₛ of the chamber.

According to one embodiment, step (iv) is performed from 100 times to 1000 times per second, preferably from 250 times to 750 times per second, more preferably from 400 times to 600 times per second. Advantageously, repeating step (iv) reduces the risk of acoustic streaming.

In a preferred configuration of this embodiment, step (iv) is performed about 500 times per second.

According to one embodiment, said method further comprises an additional step after step (iv) (named step (iv')) of sweeping the acoustic waves frequency from the second frequency f₂ to a third frequency f₃ using a sweep time ranging from 0.5 ms to 10 ms, f₂ being superior to f₃.

In a preferred configuration of this embodiment, all the frequencies in a defined domain, *i.e.* [f₁-f_{2]} then [f₃-f_{2]}, are continuously swept and in a round trip in a given sweep time.

According to one embodiment, third frequency f₃ is ranging from 0.1 MHz to 4 MHz, preferably from 0.5 MHz to 2 MHz, more preferably from 0.8 MHz to 1.5 MHz, even more preferably from 0.9 MHz to 1.1 MHz.

According to one embodiment, first frequency f₁ is equal to third frequency f₃. In this embodiment, the additional step (iv') corresponds to a return or frequency sweep using a same or different sweep time that in step (iv).

According to one embodiment, the sweep time of additional step (iv') is equal or different from the sweep time of step (iv). In a preferred configuration of this embodiment, the sweep time of additional step (iv') is equal to the sweep time of step (iv).

According to one embodiment, the sweep rate of steps (iv) and/or (iv') is ranging from 1 kHz to 10 kHz, preferably from 5 kHz to 10 kHz. Advantageously, a small sweep rate allows for more effective suppression of acoustic streaming.

According to one embodiment, the residence time of the cells in the chamber is ranging from 1 seconds to 60 seconds, preferably from 5 seconds to 30 seconds more preferably from 5 seconds to 15 seconds. The method of the invention is advantageously fast compared to conventional transduction methods wherein the transfer of nucleic acids in cells can take up from 10 minutes to several hours. Thus, in the same time as a conventional transduction method, for example 10 minutes, a much larger number of cells are modified using the method of the invention. For example, the method of the invention allows for treating (i.e. transducing or transfecting) 50 000 to 150 000 cells per minute.

According to one embodiment, the nucleic acids are carried in a viral vector or a microvesicle. In this embodiment, the method of the invention is a transduction method.

In a specific configuration of this embodiment, the viral vector is selected from adenovirus, paramyxovirus, or a retrovirus such as a lentivirus. Retroviruses are characterized by their ability to retrotranscribe their RNA genome into a cDNA copy, which is then stably integrated into the host cell genome. In this embodiment, the method for introducing foreign nucleic acids into cells is a method for performing transduction of cells in which the viral vector carries the nucleic acid into a cell, either by entering the cell itself or by inserting the nucleic acid into the cell.

Examples of retroviruses comprise simple or complex retroviruses (e.g. lentiviruses). They contain two copies of positive-stranded RNA with an associated viral reverse transcriptase (RT) located within an internal core with structural and enzymatic proteins, including the nucleocapsid (NC), capsid (CA), integrase (IN), and protease (PR). Said internal core is surrounded by an outer protein layer containing a matrix (MA) protein, which is encapsulated in an envelope glycoprotein (ENV)-studded.

Lentiviral and retroviral gene delivery vectors exploit aspects of retrovirus replication to provide stable integration of the desired nucleic acid sequence. Whereas transfection of foreign nucleic acids results only in transient transgene expression, the activity of the viral integrase in retroviral and lentiviral-based systems allows for stable integration of the foreign transgene, which is then inherited and continuously expressed over repeated cell divisions. A key feature of both lentiviral and retroviral vectors is that they produce replication-defective, or self-inactivating, particles. This allows for delivery of the desired sequence, without continued viral replication in the target cells.

According to an alternative embodiment, the nucleic acids are naked nucleic acids (herein also called plasmids), *i.e.* they are not contained or carried in a viral vector. In this embodiment, the method for introducing foreign nucleic acids into cells is a method for performing transfection of cells in which the naked nucleic acids enter the cells.

According to one embodiment, the cells are selected among Chinese hamster ovary (CHO) cells, NSO hybridoma cells, baby hamster kidney (BHK) cells, human cells, regulatory T- cells, helper T-cells, cytotoxic T-cells, memory T-cells, effector T-cells, gamma delta T- cells, Jurkat T-cells, CAR-T cells, B cells, or NK cells, peripheral blood mononuclear cells (PBMCs), HEK293T, algae, plant cells, Tumor Infiltrating Lymphocytes (TIL), or bacteria.

According to one embodiment, the cells and the nucleic acids are suspended in the same fluidic medium, in particular in a buffer. In a preferred configuration of this embodiment, the medium is Roswell Park Memorial Institute medium (RPMI), with 10% FBS fetal bovine serum and 1% penicillin/streptomycin.

The buffer is chosen according to its acoustic and fluid qualities and its compatibility with the cells and nucleic acids. The buffer comprising the nucleic acids is designed so as to remain in the focal plane of the chamber, for example, by adding a solute or a colloidal suspension for reducing the turbulence or thinning it by adding a solvent such as water. The buffer is also designed to reduce the uncontrolled exchange of chemical and particulate type with the suspension.

According to one embodiment, the buffer may be, but not limited to, CPD (citrate-phosphate-dextrose), SSP+, RPMI medium, LB (Lysogeny Broth), DMSO (Dimethyl sulfoxide), Methylcellulose, HEPES (4-(2-HydroxyEthyl)-1-PiperazineEthaneSulfonic acid), PBS (Phosphate-Buffered Saline), CMRL medium (comprising phenol red), DMEM (Dulbecco's Modified Eagle Medium), or a mixture thereof.

According to one embodiment, the buffer is configured not to involuntarily alter the cells that have to be deviated. For example, so as not to involuntarily alter the cells, unwanted chemical compounds for cells are avoided, the osmotic force applied to the cells may be reduced in the buffer and/or an adequate pH value range of cells is retained.

According to one embodiment, the multiplicity of infection is ranging from 0.5 to 30, preferably from 0.5 to 20, more preferably from 0.5 to 5, even more preferably from 0.7 to 1. A method for transduction with a low multiplicity of infection is particularly advantageous as less viral vectors are used for an enhanced transfer efficiency. As said, viral vectors can be hard to provide and are an expensive commodity, it is particularly interesting to avoid the waste of viral vectors by having a number of viral vectors as close as possible to the number of cells. For example, compared to a typical transduction method, the method of the invention exhibits an increase of at least 20% of transduction efficiency with 10 times less viral vectors.

In a preferred configuration of this embodiment, the multiplicity of infection is 1, *i.e.* 1 viral vector is used per cell.

According to one embodiment, the number of cells treated by the method of the invention is ranging from 50 000 cells/minute to 150 000 cells/minute. In a specific configuration of this embodiment, the number of cells treated by the method of the invention is ranging from 50 000 cells/minute to 150 000 cells/minute at an injection rate of 0.5 ml/min concerning the suspension of cells. Thus, the method of the invention is readily scalable for treating large numbers of cells, as needed in clinical protocols.

According to one embodiment, the suspension of cells and/or the suspension comprising foreign nucleic acid further comprise additives, such as for example polyhexadimethrine bromide, a polypeptide consisting of three functional domains derived from human fibronectin protein (C-domain, H-doman, and CS-1 site), lipofectin, lipofectamine, or cationic peptides such as protamine The first additive is used to increase the efficiency of transduction of certain cells with retrovirus (and lentivirus) in cell culture, and is typically used in amounts of 10 pg/mL or less. The second additive enhances the efficiency of retroviral and lentiviral mediated transduction in hematopoietic cells including hematopoietic stem cells and terminally differentiated cells such as primary T cells and macrophages.

According to one embodiment, the acoustic waves generated are volumetric acoustic waves, surface acoustic waves, standing surface acoustic waves, multi-dimensional acoustic waves or standing acoustic waves.

According to one embodiment, only one acoustic pressure node is created within the chamber upon applying the acoustic field, preferably said acoustic pressure node is located at the center of the chamber.

In a specific configuration of this embodiment, said acoustic pressure node is located at mid-height (e/2, e being the thickness of the chamber).

According to one embodiment, the power applied to the acoustic wave generator is ranging from 0.2 W to 50 W, preferably from 0.2 W to 25 W, more preferably from 0.5 W to 10 W, even more preferably from 1 W to 4 W.

In a preferred configuration of this embodiment, the power applied to the acoustic wave generator is 2W.

According to one embodiment, the injection rate and the collecting rate are ranging from 0.1 ml/min to 100 ml/min. Both rates are dependent on the species (cells, viral vectors) involved.

In a specific configuration of this embodiment, the collecting rate is ranging from 0.1 ml/min to 10 ml/min, more preferably from 0.5 ml/min to 5 ml/ min, most preferably from 0.5 ml/min to 1.5 ml/min.

In a specific configuration of this embodiment, the injection rate of the cells suspension and/or the injection rate of the suspension comprising foreign nucleic acids are ranging from 0.1 ml/min to 100 ml/min. Preferably, the injection rate of the cells suspension and/or the injection rate of the suspension comprising foreign nucleic acids are ranging from 0.1 ml/min to 10 ml/min, more preferably from 0.5 ml/min to 5 ml/ min, most preferably from 0.5 ml/min to 1 ml/min.

In a specific configuration of this embodiment, wherein the acoustophoresis device comprises two inlets (first inlet and second inlet), the injection rates of the suspension comprising cells and the suspension comprising foreign nucleic acids are equal.

In another specific configuration of this embodiment, wherein the acoustophoresis device comprises three inlets, *i.e.* a first central inlet and two second peripheral inlets, the injection rate of the suspension comprising foreign nucleic acids is superior to the injection rates at the two second peripheral inlets. In particular, the ratio between the injection rate of the suspension comprising foreign nucleic acids and the injection rate at each of the two second peripheral inlets is ranging from 1.2 to 2, preferably from 1.6 to 1.9, more preferably equal to 1.8.

The invention also relates to a method for performing transduction of cells, comprising:
i. providing an acoustophoresis device comprising:
   - a chamber configured to be associated with at least one acoustic wave generator for generating acoustic waves within the chamber;
   - at least one acoustic wave generator configured to generate acoustic waves within the chamber;
   - at least two inlets in fluid communication with the chamber, said inlets comprising a first inlet and at least one second inlet;
   - at least two outlets in fluid communication with the chamber, said outlets comprising a first outlet and at least one second outlet;
ii. injecting a suspension of viral vectors comprising nucleic acids in the chamber at the first inlet and injecting a cell suspension in the chamber at the at least one second inlet;
iii. applying an acoustic field by generating acoustic waves inside the chamber;
iv. sweeping the acoustic waves frequency from a first frequency f₁ to a second frequency f₂ using a sweep time ranging from 1 ms to 100 ms, f₂ being superior to f₁;
v. collecting a suspension comprising cells modified by nucleic acids at the first outlet.

All the preceding embodiments and advantages apply to this transduction method.

The transduction method uses an acoustophoresis device as described hereafter to perform transduction of cells.

According to one embodiment, first frequency f₁ and second frequency f₂ are each ranging from 0.1 MHz to 4 MHz.

According to one embodiment, said method further comprises an additional step after step (iv) (named step (iv')) of sweeping the acoustic waves frequency from the second frequency f₂ to a third frequency f₃ using a sweep time ranging from 0.5 ms to 10 ms, f₂ being superior to f₃.

According to one embodiment, the cells are selected among Chinese hamster ovary (CHO) cells, NSO hybridoma cells, baby hamster kidney (BHK) cells, human cells, regulatory T- cells, helper T-cells, cytotoxic T-cells, memory T-cells, effector T-cells, gamma delta T- cells, Jurkat T-cells, CAR-T cells, B cells, or NK cells, peripheral blood mononuclear cells (PBMCs), algae, plant cells, HEK293T, Tumor Infiltrating Lymphocytes (TIL), or bacteria.

The invention also relates to a method for performing transfection of cells, comprising:
i. providing an acoustophoresis device comprising:
   - a chamber configured to be associated with at least one acoustic wave generator for generating acoustic waves within the chamber;
   - at least one acoustic wave generator configured to generate acoustic waves within the chamber;
   - at least two inlets in fluid communication with the chamber, said inlets comprising a first inlet and at least one second inlet;
   - at least two outlets in fluid communication with the chamber, said outlets comprising a first outlet and at least one second outlet;
ii. injecting a suspension of plasmids in the chamber at the first inlet and injecting a cell suspension in the chamber at the at least one second inlet;
iii. applying an acoustic field by generating acoustic waves inside the chamber;
iv. sweeping the acoustic waves frequency from a first frequency f₁ to a second frequency f₂ using a sweep time ranging from 1 ms to 100 ms, f₂ being superior to f₁;
v. collecting a suspension comprising cells modified by plasmids at the first outlet.

All the preceding embodiments and advantages apply to this transfection method.

The transfection method uses an acoustophoresis device as described hereafter to perform transfection of cells.

In conventional transfection methods, pores are opened in the cell membranes of the cells to permit nucleic acids to enter the cells. Typically, the cells are subjected to electroporation, where the cells are exposed to an electric field to increase the permeability of the cell membrane, or sonoporation, where the cells are exposed to ultrasounds to induce pore formation in the cell membrane.

The present invention also relates to cells obtained by any one of the methods of the invention.

The present invention also relates to an acoustophoresis device for introducing foreign nucleic acids into cells comprising:
- a chamber configured to be associated with at least one acoustic wave generator for generating acoustic waves within the chamber, said chamber extending along a longitudinal axis;
- at least one acoustic wave generator configured to generate acoustic waves within the chamber;
- at least two inlets in fluid communication with the chamber, said inlets comprising a first inlet for introducing a suspension comprising foreign nucleic acids in the chamber, and at least one second inlet for introducing a cell suspension in the chamber;
- at least two outlets in fluid communication with the chamber, said outlets comprising a first outlet for collecting a suspension comprising cells modified by foreign nucleic acids and at least one second outlet;
wherein the at least one acoustic wave generator is configured to sweep the acoustic waves frequency from a first frequency f₁ to a second frequency f₂ using a sweep time ranging from 1 ms to 100 ms, f₂ being superior to f₁.

The acoustophoresis device is configured to implement any of the methods of the invention.

According to one embodiment, the at least two inlets are located on one end of the chamber and the at least two outlets are located on the other end along the longitudinal axis of the chamber.

According to one embodiment, the first inlet and the first outlet are aligned with the focusing plane created at mid-height in the chamber upon the application of the acoustic field. Said first inlet and said first outlet are referred hereafter as "central inlet" and "central outlet" respectively.

According to one embodiment, the at least one second inlet and the at least one second outlet are not aligned with the focusing plane created at mid-height in the chamber upon the application of the acoustic field. Said at least one second inlet and said at least one second outlet is referred to hereafter as "peripheral inlet" and "peripheral outlet" respectively.

According to one embodiment, the acoustophoresis device comprises two inlets, a first inlet and a second inlet, and two outlets, a first outlet and a second outlet. Said first inlet and said first outlet are aligned with the focusing plane and called "central inlet" and "central outlet" respectively, whereas said second inlet and said second outlet are not aligned with the focusing plane and called "peripheral inlet" and "peripheral outlet" respectively.

According to one embodiment, the acoustophoresis device comprises three inlets, *i.e.* a first central inlet and two second peripheral inlets, and three outlets, *i.e.* a first central outlet and two second peripheral outlets. Said first inlet and said first outlet are aligned with the focusing plane and called "central inlet" and "central outlet" respectively, whereas said second inlets and said second outlets are not aligned with the focusing plane and called "peripheral inlets" and "peripheral outlet" respectively. Furthermore, said second inlets are each located on either side of the longitudinal axis of the chamber, and said second outlets are each located on either side of the longitudinal axis of the chamber. In other words, said second inlets are located opposite from each other according to the longitudinal axis, and second outlets are located opposite from each other according to the longitudinal axis.

The acoustic wave generator is configured to emit an acoustic wave, said acoustic wave having a defined amplitude and a defined frequency, said acoustic wave generator being located between the first inlet and the first outlet. The acoustic waves allow the displacement of cells from their initial suspension to the suspension comprising foreign nucleic acids, resulting in an enhanced transduction or transfection reaction due to confinement of cells with foreign nucleic acids in the same layer of fluids.

According to one embodiment, the acoustic wave generator is configured to generate bulk acoustic waves (BAW), *i.e.* volume acoustic waves propagating in 3 dimensions, *i.e.* acoustic waves propagating between the acoustic wave generator and the chamber through the bulk of the coupling material. Generating bulk acoustic waves in the chamber advantageously allows for treatment (i.e. transduction or transfection) of big volumes of cells suspension, which allows for an efficient and easy to implement temporary coupling between the acoustic wave generator and the chamber.

The at least one acoustic wave generator does not generate surface acoustic waves (SAW), stationary surface acoustic waves (SSAW), or travelling surface acoustic waves (TSAW). Surface acoustic waves (SAW) refer to acoustic waves propagating between the acoustic wave generator and the chamber along a surface. Surface acoustic waves (SSAW) refer to SAW oscillating in time but whose peaks do not travel in space. Travelling surface acoustic waves (TSAW) refer to SAW whose peaks do travel in space. The use of SSAW limits the capacity for temporary coupling between the acoustic wave generator and the chamber, and the volume processed, making it a poor candidate for the production of contamination sensitive suspensions. The use of travelling waves (e.g TSAW) renders the device of the invention ineffective.

According to one embodiment, the acoustic wave generator is a piezoelectric transducer. The transducer may be made of a ceramic, such as lead zirconate, titanate, potassium niobate, or sodium tungstate; a crystal, such as quartz, tourmaline, or gallium phosphate; III-V or II-VI semiconductors such as gallium nitride or zinc oxide; polymers such as polyvinylidene fluoride, polyvinylidene chloride, or polyimide; or a mixture thereof.

In a specific configuration of this embodiment, the transducer is a longitudinal resonator which allows for an efficient and easy to implement temporary coupling between the transducer and the chamber.

According to one embodiment, the acoustic wave generator is configured to emit acoustic waves with a frequency ranging from 0.1 MHz to 4 MHz, preferably from 0.5 MHz to 2 MHz, more preferably from 0.8 MHz to 1.5 MHz, even more preferably from 0.9 MHz to 1.1 MHz. The frequency of the emitted waves is chosen depending on the particles to be moved in the suspension, in particular said frequency is selected based on the particle size to be deflected.

According to one embodiment, the acoustic wave generator is external to the chamber.

In one embodiment, the acoustic wave generator generates an acoustic force field over the thickness, not over the width of the chamber. This embodiment is particularly advantageous as it allows the formation of layers of fluid along the thickness of the chamber.

In one embodiment, the acoustic waves have an incident angle to the longitudinal axis of the chamber ranging from 85° to 95°, *e.g.* from 89° to 91°.

In one embodiment, the acoustic waves have an incident angle to the longitudinal axis of the chamber of substantially 90°.

According to one embodiment, the acoustic wave generator is coupled longitudinally to a wall of the chamber, in particular to a longitudinal wall. In other words, the acoustic wave generator extends along the same longitudinal axis as the chamber.

According to one embodiment, the acoustic wave generator is temporarily coupled to the chamber. Temporary coupled means removably coupled. This embodiment is particularly advantageous as it allows using a disposable chamber with a non-disposable acoustic wave generator, thus ensuring the sterility of the chamber, which remains a primordial point in the handling of biological objects. The acoustic wave generator is not glued to the chamber using, for example, an epoxy resin.

In a preferred configuration of this embodiment, the temporary coupling is provided by the use of a coupling layer between a wall of the chamber and the acoustic wave generator. This advantageously prevents the presence of air bubbles between the chamber and the acoustic wave generator, ensuring an improved contact between the chamber and the acoustic wave generator. The coupling layer allows for a much more efficient transmission of the acoustic energy between the acoustic wave generator and the chamber.

According to one embodiment, said coupling layer is made from thermoplastic elastomers, thermoplastic polyurethanes or silicone.

According to one embodiment, said coupling layer is made of oil or a mixture comprising oil.

In particular, examples of coupling layers include but are not limited to: a liquid or a gel, such as for example water, hydrogel, vegetable oil, mineral oil, grease, polymer, derivative thereof, or mixture thereof. The coupling element should be as thin as possible, homogeneous, i.e. without any bubbles or microbubbles within said coupling elements or on contact surfaces.

In one embodiment, the chamber extends along a longitudinal axis, has a cross section with a width measured along a first transverse axis and a thickness measured along a second transverse axis perpendicular to the first transverse axis, the width being greater than or equal to the thickness, the chamber having first and second walls along the second transverse axis.

According to one embodiment, the chamber has a rectangular shape, cylindrical shape or other shape. In a preferred configuration of this embodiment, the chamber is a rectangular parallelepiped characterized by a length (along the longitudinal axis A1), a width and a thickness, this configuration is particularly advantageous as it is easier to apply an acoustic wave without perturbations to a parallelepiped than any other shape. The chamber can also be called "channel" herein.

According to one embodiment, the chamber is not a microfluidic channel, thus the device of the invention is not a microfluidic device. The use of a non-microfluidic channel allows to significantly and easily increase the flow rate in the chamber, and so reduce the processing time for a given volume, while keeping the shear rate low enough for the particles not to be damaged during the process, therefore allowing for the use of this device to produce cell therapy treatments.

According to one embodiment, the chamber has a width ranging from 1 mm to 50 mm, preferably from 5 mm to 20 mm, more preferably from 5 mm to 15 mm, said width being the diameter in case of the chamber is a cylinder.

According to one embodiment, the chamber has a thickness ranging from 150 µm to 2 mm, preferably from 300 µm to 780 µm.

According to one embodiment, the chamber has a length ranging from 5 cm to 20 cm, preferably from 5 cm to 15 cm.

In one embodiment, the acoustic waves have a wavelength λ and the thickness of the chamber is substantially equal to a multiple of λ/4.

According to one embodiment, the chamber comprises internal walls made of a material having an acoustic impedance superior to the acoustic impedance of the fluid flowing in said chamber.

In a specific configuration of this embodiment, the chamber or the internal walls of said chamber are made of a material selected among the group of organic polymers or inorganic polymers; metals; gels, such as for example hydrogel; glass, such as for example, fused quartz, borosilicate glass; crystals, such as for example silicon; ceramics, such as for example, silicon carbide; resins; derivatives thereof, or a mixture thereof. The material of the chamber or internal walls thereof should be easy to sterilize after use.

Examples of organic polymers include but are not limited to: poly(methyl methacrylate) (PMMA) polyurethane, silicone, polyethylene, polymethylpentene, polystyrene, polycarbonate, polydimethylsiloxane, medical grade polymers, or disposable plastic.

Examples of metals include but are not limited to: steel or stainless steel. Advantageously, stainless steel allows a better transmission of acoustic energy to the fluid in the chamber. Furthermore, a steel wall can play the role of a reflector in the device.

Advantageously, PMMA has an acoustic impedance twice as high as that of fluids, stainless steel has an acoustic impedance 40 times higher than that of fluids. Furthermore, PMMA is advantageously compatible with all cells, is optically transparent, i.e. allows to see the interior of the chamber, thus the fluids herein.

The device of the invention does not comprise a reflector configured to reflect the acoustic waves in the chamber. The device of the invention uses a layer of free air at the outside of the chamber as a potential reflector.

According to one embodiment, the chamber is sterile.

According to one embodiment, the device is disposable. The device is usually in contact with fluids that may be contaminated. The disposability ensures a good hygiene, regulatory compliance and saves time. In this embodiment, the system is changed after every use.

According to an alternative embodiment, the device and all its parts are disinfectable. In this embodiment, the device can be disinfected using a cleaning bath, a disinfectant wipe, or any other means known by one skilled in the art.

In one embodiment, the device is a portable device. In this embodiment, the dimensions of the device and the connections between each part of said device allows an easy transportation of said device.

According to one embodiment, the device further comprises at least one pumping means connected to the chamber. The pumping means are configured to manage the flow of fluid within the device, *i.e.* within the chamber. In particular, the pumping means able to apply a controlled and constant flow rate at each inlet of the device and/or in the chamber, and advantageously allows maintaining the sterility of the chamber. In this embodiment, the pumping means is a mechanical pump, preferably the pumping means is a peristaltic pump. In a specific configuration, each inlet is connected to one pumping means.

According to one embodiment, the acoustophoresis device further comprises a flow rate sensor configured to measure and/or control the flow rate within the chamber. This allows checking and tune the flow rate so that the flow of fluid within the chamber is as smooth as possible in order to respect the Poiseuille's law.

In a specific configuration of this embodiment, the flow rate sensor is a contactless flow rate sensor, *i.e.* said sensor does not have any contact with the fluid in the chamber. This is particularly advantageous as a contactless flow rate measurement/monitoring ensures the sterility of the device and the fluid herein.

According to one embodiment, the acoustophoresis device further comprises a pressure sensor.

According to one embodiment, the acoustophoresis device further comprises a concentration sensor to measure the concentration of cells and/or nucleic acids in the chamber.

According to one embodiment, the device further comprises an electronic control unit configured to monitor the flow rate of the fluid in the chamber on the basis of the recovered data from the pressure sensor, the concentration sensor and/or the flow rate sensor.

According to one embodiment, the device further comprises a temperature sensor and a temperature control system configured to monitor and control the temperature of the fluid flowing inside the chamber. In particular, the temperature control system is configured to ensure that said temperature stays below the threshold. This embodiment is particularly advantageous at it ensures that the chamber does not heat up during activation of the acoustic wave generator (that produces heat), thus that fluids in the chamber does not heat up. This protects the cells and nucleic acids in the chamber from deterioration due to an excessive heat or an unexpected activation.

In a specific configuration of this embodiment, said threshold is 35°C, preferably 30°C, more preferably 25°C, even more preferably 20°C.

In a specific configuration of this embodiment, said temperature control system is a cooling system such as for example a Peltier cooling system or a water-cooling system.

According to one embodiment, the acoustophoresis device comprises a plurality of acoustic wave generators, preferably the device comprises 2, 3, or 4 acoustic wave generators. This embodiment is advantageous as it allows a better power distribution along the chamber. The acoustic wave generators are preferably aligned along the longitudinal axis (A1) of the chamber.

In a specific configuration of this embodiment, the acoustic waves emitted by said acoustic wave generators may be identical or different.

The use of a plurality of acoustic wave generators is advantageous when the fluid flows at high velocity. In the first case, the flight time under the acoustic wave generator decreases as the fluid velocity increases. This may require a greater number of acoustic wave generators to be used in order to achieve focusing.

When a plurality of acoustic wave generators is used, at least one of them may generate an acoustic wave along the width of the chamber.

In one embodiment, the device comprises a plurality of chambers, each one connected to an acoustic wave generator. Said chambers are arranged in parallel. In this embodiment, several volumes can be treated simultaneously with acoustic waves in the chambers before being collected, ensuring that the large volumes of cells can be treated in a short time.

In one embodiment, the fluidic connection between the different parts of the device comprises any means known by one skilled in the art, such as flexible manifolds or tubes and clamps or valves. Their representations in the drawings are not representative of their dimensions and positions.

### BRIEF DESCRIPTION OF THE DRAWINGS

**Figure 1** is a schematic representation of the application of an acoustic field on cells within a chamber.
**Figure 2A** is a schematic representation of an acoustophoresis device 1 according to a first embodiment of the invention.
**Figure 2B** is a schematic representation of the acoustophoresis device 1 illustrated in Figure 2A, wherein cells 2 and foreign nucleic acids flow within the chamber 11.
**Figure 3A** is a schematic representation of an acoustophoresis device 1 according to a second embodiment of the invention.
**Figure 3B** is a schematic representation of the acoustophoresis device 1 illustrated in Figure 3A, wherein cells 2 and foreign nucleic acids flow within the chamber 11.
**Figure 4A** is a schematic representation of an acoustophoresis device 1 according to a third embodiment of the invention.
**Figure 4B** is a schematic representation of an acoustophoresis device 1 according to a fourth embodiment of the invention.
**Figure 5A** illustrates the sweeping of acoustic waves frequency from a first frequency of 0.1 MHz to a second frequency of 1 MHz using a sweep time of 1 ms.
**Figure 5B** is a zoom of Figure 5A representing only the first 0.2 ms of the sweep, in order to visualize more easily the effect of a frequency sweep.

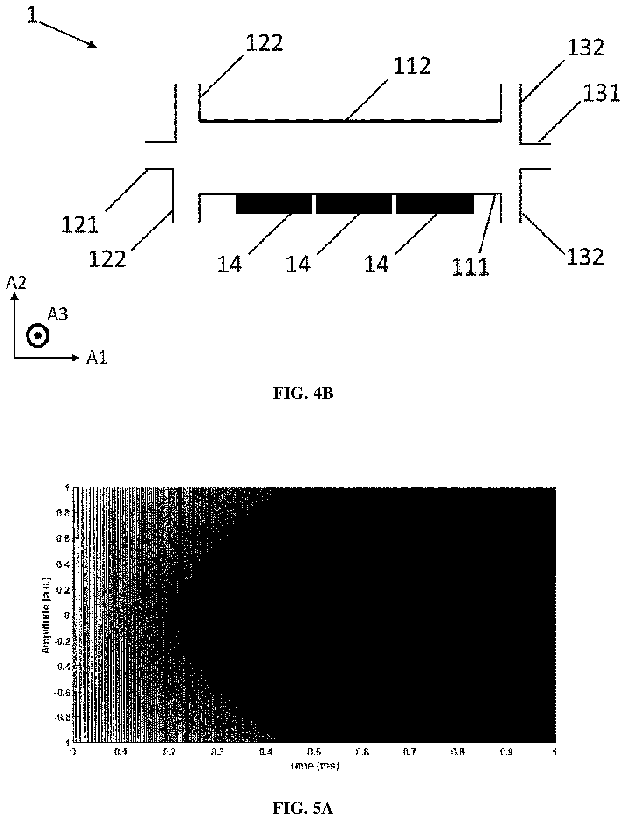

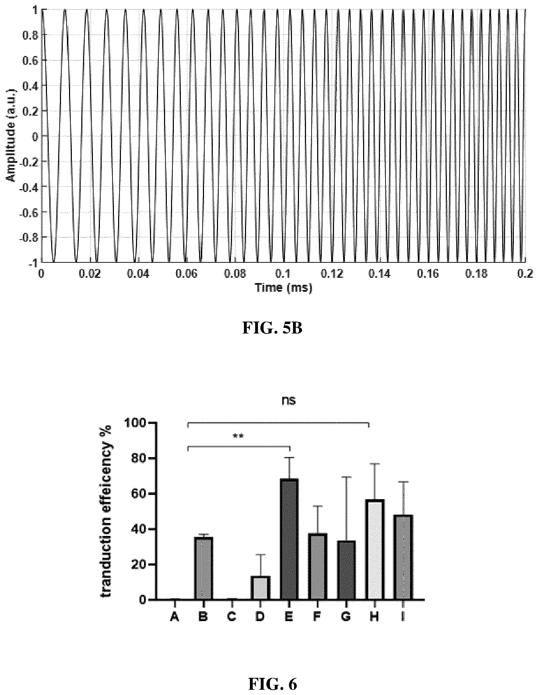

**Figure 5** illustrates the sweeping of acoustic waves frequency from a first frequency of 0.9 MHz to a second frequency of 1.1 MHz using a sweep time of 1 ms.
**Figure 6** is a statistical analysis of the transduction efficiency by flow cytometry showing the percentage of the GFP positive cells in different conditions, A: control sample, cells without acoustic in static condition in the tissue culture well plates with no virus, B: cells in static condition, no acoustic in the tissue culture well plates with viral vector, C: cells in a flow channel with acoustic without virus, D: cells collected from lateral outlets 132 on top of a flow channel with acoustic power of 1W and in presence of viral vectors, E: cells collected from outlet 131 of the flow channel with acoustic power of 1W and in presence of viral vectors, F: cells collected from lateral outlets 132 on the bottom of a flow channel with acoustic power of 1W and viral vectors, G: cells collected from lateral outlets 132 on top of a flow channel with acoustic power of 2W and viral vectors, H: cells collected from outlet 131 in the flow channel with acoustic power of 2W and viral vectors, I: cells collected from lateral outlets 132 on the bottom of a flow channel with acoustic power of 2W and viral vectors. The n= 3 independent biological experiment. The percentage of transduction efficiency on samples from central outlets (E and H) compared to the control value (B) has been quantified by conducting the T-test; the P-value of ≥0.05 corresponds to ns, 0.01 to 0.05 ^{∗} and 0.001 to 0.01 ^{∗∗}.

### ILLUSTRATIVE EMBODIMENTS OF THE INVENTION

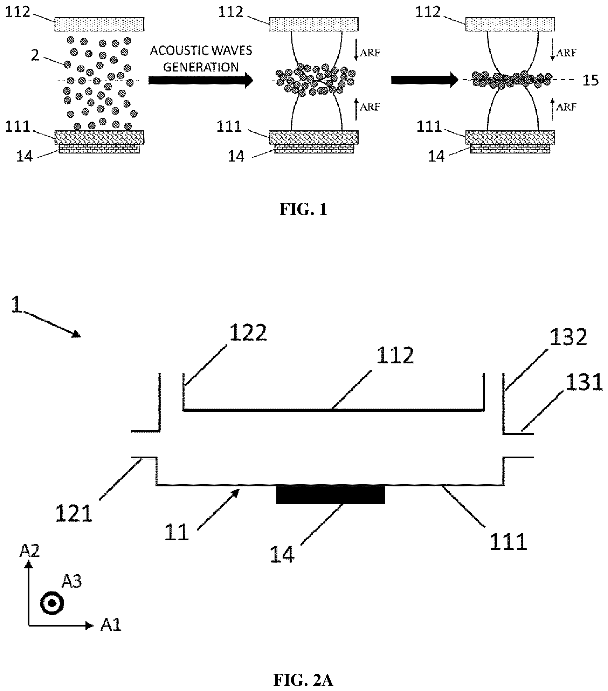

The application of an acoustic field on cells 2 within a chamber between a transfer wall 111 and an opposite wall 112 of said chamber is shown in Figure 1. The transfer wall 111 and the opposite wall are longitudinal walls of said chamber, i.e. both walls extend along the same longitudinal axis of said chamber. The transfer wall is coupled to an acoustic wave generator 14 and the opposite wall can play the role of a reflector. As shown in Figure 1, the application of an acoustic field on cells 2 within said chamber induces a movement of the cells 2, allowing to deflect and gather said cells 2 at a node of said acoustic field.

In this example, ultrasonic waves are generated in a chamber between a reflector (here the opposite wall 112) and the transfer wall 111 associated with an acoustic wave generator 14. This enables the creation of an acoustic pressure node in the center of the chamber (depending on the chosen frequency at which the acoustic wave generator 14 operates), a focusing plane 15 is located on the acoustic pressure node, and acoustic radiation forces (ARF) are generated in the chamber. The ARF push the cells 2 towards the pressure node with a force of up to a hundred times gravity equivalent. The cells 2 suspended in the fluid will then migrate to the sound pressure node and can then remain trapped at the focusing plane 15.

This is particularly advantageous as it allows confinement of the cells 2 within a fluid at a certain location of the chamber, in particular at the focusing plane 15, without any mechanical force, pressure or invasive procedure that could damage said cells 2.

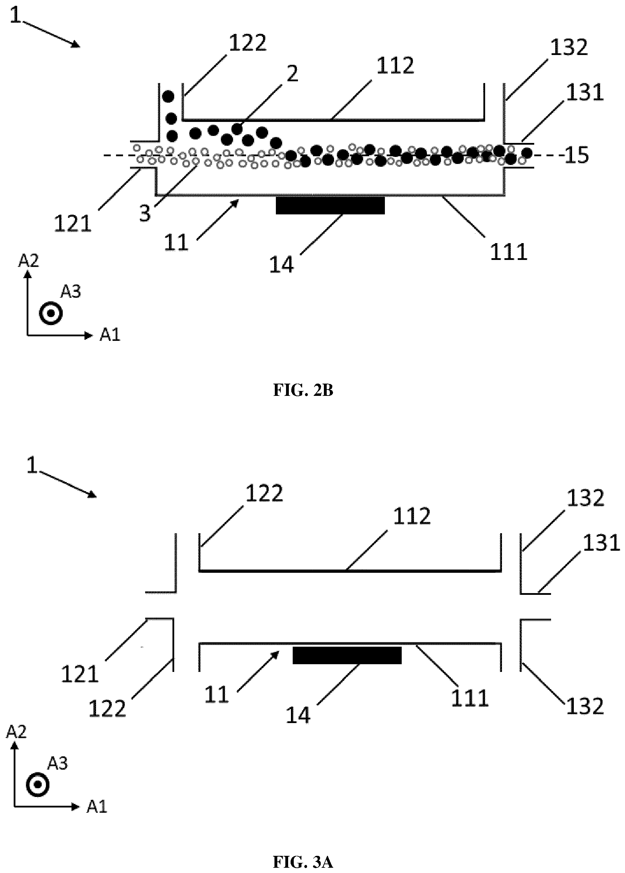

In the first embodiment shown in Figure 2A and Figure 2B, the acoustophoresis device 1 comprises:
- a chamber 11 associated with an acoustic wave generator 14 for generating acoustic waves within the chamber 11, said chamber extending along a longitudinal axis A1;
- an acoustic wave generator 14 longitudinally aligned with the longitudinal axis A1 of the chamber 11;
- two inlets (121, 122) in fluid communication with the chamber 11, said inlets (121, 122) comprising a first inlet 121 for introducing a suspension comprising foreign nucleic acids 3 in the chamber 1, and a second inlet 122 for introducing a suspension comprising cells 2 in the chamber 1;
- two outlets (131, 132) in fluid communication with the chamber 1, said outlets (131, 132) comprising a first outlet 131 for collecting a suspension comprising cells modified by foreign nucleic acids and a second outlet 132.

The chamber extends along a longitudinal axis A1, has a first transverse axis A2 and a second transverse axis A3 perpendicular to the first transverse axis A2, the width being greater than or equal to the thickness, the chamber having first and second walls along the second transverse axis. The length of the chamber is measured along the longitudinal axis A1, the width is measured along the first transverse axis A2 and the thickness is measured along the second transverse axis A3. The chamber comprises a transfer wall 111 coupled with the acoustic wave generator 14 and an opposite wall 112 facing the transfer wall.

The chamber 11 can be divided into 3 main zones: the injection zone comprising the inlets (121, 122), the active zone where the acoustic waves are generated and the exit zone comprising the outlets (131, 132). The active zone is designed to optimize the acoustic efficiency in a controlled way through the choice of materials, the thickness of the layers and the width of the chamber11. The active zone length ranges from 1 cm to 20 cm, preferably from 1 cm to 10 cm, more preferably from 1 cm to 5 cm.

In this embodiment, the acoustophoresis device 1 comprises two inlets (121, 122) and two outlets (131, 132). The longitudinal axis of the first inlet 121 and the first outlet 131 are aligned with the longitudinal axis A1 of the chamber 11 and with the focusing plane 15 located at the center of the chamber 11, *i.e.* at about mid-height of said chamber 11. The longitudinal axis of the second inlet 122 and the second outlet 132 are aligned. Said axis are perpendicular to the longitudinal axis A1 of the chamber 11, *i.e.* aligned with the first transverse axis A2.

The foreign nucleic acids 3 suspension is injected at the first inlet 121 with a laminar flow and the cells 2 suspension is injected at the second inlet 122 with a laminar flow, thus forming two layers of fluid flowing through the chamber 11 without turbulence. Upon application of an acoustic field generated by the acoustic wave generator 14, the cells 2 are deflected from their initial suspension to the central layer of fluid, *i.e.* to the suspension comprising foreign nucleic acids 3, as the ARF push the cells 2 towards the focusing plane 15 with a force larger than the combined effect of fluid drag force and gravitational force. Due to their respective sizes, only the cells 2 are deflected while the flow of the foreign nucleic acids remains unperturbed. As the cells 2 and foreign nucleic acids 3 gather in the same layer of fluid, said foreign nucleic acids 3 are transferred to the cells 2, resulting in modified cells. Said modified cells are collected at the first outlet 131.

This embodiment is particularly advantageous as this forced confinement of cells 2 and foreign nucleic acids 3 in the same layer of fluid greatly encourages transfer of said foreign nucleic acids 3 to the cells 2.

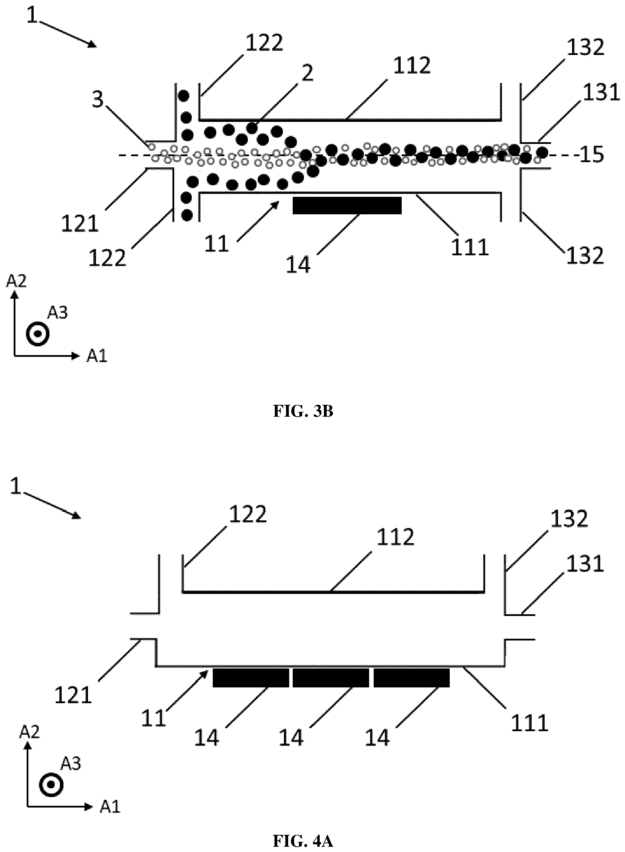

In the second embodiment shown in Figure 3A and Figure 3B, the acoustophoresis device 1 comprises:
- a chamber 11 associated with an acoustic wave generator 14 for generating acoustic waves within the chamber 11, said chamber extending along a longitudinal axis A1;
- an acoustic wave generator 14 longitudinally aligned with the longitudinal axis A1 of the chamber 11;
- three inlets (121, 122) in fluid communication with the chamber 11, said inlets (121, 122) comprising a first inlet 121 for introducing a suspension comprising foreign nucleic acids 3 in the chamber 1, and two second inlets 122 for introducing a suspension comprising cells 2 in the chamber 1;
- three outlets (131, 132) in fluid communication with the chamber 1, said outlets (131, 132) comprising a first outlet 131 for collecting a suspension comprising cells modified by foreign nucleic acids and two second outlets 132.

The chamber extends along a longitudinal axis A1, has a first transverse axis A2 and a second transverse axis A3 perpendicular to the first transverse axis A2, the width being greater than or equal to the thickness, the chamber having first and second walls along the second transverse axis. The length of the chamber is measured along the longitudinal axis A1, the width is measured along the first transverse axis A2 and the thickness is measured along the second transverse axis A3. The chamber comprises a transfer wall 111 coupled with the acoustic wave generator 14 and an opposite wall 112 facing the transfer wall.

In this embodiment, the acoustophoresis device 1 comprises three inlets (121, 122) and three outlets (131, 132). The longitudinal axis of the first inlet 121 and the first outlet 131 are aligned with the longitudinal axis A1 of the chamber 11 and with the focusing plane 15 located at the center of the chamber 11, *i.e.* at about mid-height of said chamber 11. The longitudinal axis of the second inlets 122 and the second outlets 132 are aligned. Said axis are perpendicular to the longitudinal axis A1 of the chamber 11, *i.e.* aligned with the first transverse axis A2. Furthermore, said second inlets 122 are each located on either side of longitudinal axis A1 of the chamber, and said second outlets 132 are each located on either side of longitudinal axis A1 of the chamber. In other words, said second inlets 122 are located opposite from each other according to the longitudinal axis A1, and second outlets 132 are located opposite from each other according to the longitudinal axis A1.

The foreign nucleic acids 3 suspension is injected at the first inlet 121 with a laminar flow and two cells 2 suspensions, preferably the same, are injected at the second inlets 122 with a laminar flow, thus forming three layers of fluid flowing through the chamber 11 without turbulence. Upon application of an acoustic field generated by the acoustic wave generator 14, the cells 2 are deflected from their initial suspensions to the central layer of fluid, *i.e.* to the suspension comprising foreign nucleic acids 3, as the ARF push the cells 2 towards the focusing plane 15 with a force larger than the combined effect of fluid drag force and gravitational force. Due to their respective sizes, only the cells 2 are deflected while the flow of the foreign nucleic acids remains unperturbed. As the cells 2 and foreign nucleic acids 3 gather in the same layer of fluid, said foreign nucleic acids 3 are transferred to the cells 2, resulting in modified cells. Said modified cells are collected at the first outlet 131.

This embodiment is particularly advantageous as this forced confinement of cells 2 and foreign nucleic acids 3 in the same layer of fluid greatly encourages transfer of said foreign nucleic acids 3 to the cells 2. Furthermore, having two inlets for the injection of cells 2 suspensions increase the number of cells 2 that can be modified with foreign nucleic acids.

In the third embodiment shown in Figure 4A, the acoustophoresis device 1 is identical to the acoustophoresis device 1 according to the first embodiment illustrated in Figure 2A but comprises a plurality of acoustic wave generators 14 coupled to the transfer wall of the chamber 1, said acoustic wave generators 14 being longitudinally aligned with the longitudinal axis (A1) of the chamber 11.

This embodiment is particularly advantageous as the active zone of the chamber *1, i.e.* the zone where acoustic waves are generated in the chamber 1, is lengthened. This also allows a better power distribution along the chamber 1.

In the fourth embodiment shown in Figure 4B, the acoustophoresis device 1 is identical to the acoustophoresis device 1 according to the first embodiment illustrated in Figure 3A but comprises a plurality of acoustic wave generators 14 coupled to the transfer wall of the chamber 1, said acoustic wave generators 14 being longitudinally aligned with the longitudinal axis (A1) of the chamber 11.

This embodiment is particularly advantageous as the active zone of the chamber *1, i.e.* the zone where acoustic waves are generated in the chamber 1, is lengthened. This also allows a better power distribution along the chamber 1.

### EXAMPLES

The present invention is further illustrated by the following examples.

### Example 1: Transduction efficiency with frequency sweeping

### Materials and Methods

A suspension of cells is prepared, comprising Jurkat E6 cells, RPMI media with 10% FBS and 1% penicillin/streptomycin, with a number of cells/ml of 100 000 cells/ml. Cells were used in RPMI media with 10% FBS and 1% penicillin/streptomycin.

A suspension of viral vectors comprising nucleic acids is prepared, comprising VLPs-VSV (GFP), (Virus-Like Particles-Vesicular Stomatitis Virus (green fluorescent protein)), as viral vectors in RPMI media with 10% FBS and 1% penicillin/streptomycin at a concentration of 2µl/ml.

Thus, the MOI is inferior to 1.

The transduction of cells is performed as follows:
i. an acoustophoresis device is provided, said acoustophoresis device comprising:
   - a chamber associated with an acoustic wave generator for generating acoustic waves within the chamber;
   - three inlets in fluid communication with the chamber, a first central inlet for introducing a suspension comprising foreign nucleic acids in the chamber and two second peripheral inlets for introducing a cell suspension in the chamber; and
   - three outlets in fluid communication with the chamber, a first central outlet for collecting a suspension comprising cells modified by foreign nucleic acids and two second peripheral outlets;
ii. the suspension of viral vectors is injected in the chamber at the first central inlet and the cell suspension is simultaneously injected in the chamber at the two second peripheral inlets;
iii. an acoustic field is applied to generate acoustic waves inside the chamber;
iv. acoustic waves frequency sweeping is performed, said frequency sweeping comprising 2 parts defining a sweeping cycle:
   - a first part with a starting frequency f₁ of 0.9 MHz and an ending frequency f₂ of 1.1 MHz (see fig 5A and 5B for an illustration with different frequencies), using a sweep time of 1 ms;
   - a second part called "return sweep", with a starting frequency f₂ of 1.1 MHz and an ending frequency f₁ of 0.9 MHz, using a sweep time of 1 ms;
   - total sweep time for one sweeping cycle with part one and part two is then 2 ms;
   - the signal is a continuous wave, i.e. the sweeping cycle is repeated continuously, around 500 times per second;
v. a sample comprising cells modified by nucleic acids is collected at the first central outlet.

The transduction parameters of each test are detailed in Table I below:

**Table I: Experimental protocol for samples 1-4**

| sample | cells injection rate | Viral vectors injection rate | Acoustic exposure time | Power (W) |
|---|---|---|---|---|
| 1 | - | - | - | 0 |
| 2 (control) | 0.5 ml/min | No viral vectors | 30 seconds | 1 |
| 3 | 0.5 ml/min | 0.5 ml/min | 30 seconds | 1 |
| 4 | 0.5 ml/min | 0.5 ml/min | 30 seconds | 2 |

Table I shows the experimental protocol for both static conditions (sample 1) and continuous flow acoustic conditions (samples 2-4). Static conditions refer to conditions wherein the cells and viruses were incubated in the cell culture dish, whereas continuous flow acoustic conditions refer to conditions wherein cells and viruses were injected inside the channel of the acoustophoresis device and exposed to acoustic waves. The acoustic exposure time represents the time during which the cells are exposed to the acoustic waves (roughly the time it takes for the cells to go across the active area of the channel at a flow rate of 0.5 ml/min).

The first sample corresponds to static conditions wherein the transduction was performed using the 24 wells tissue culture plate (no acoustic waves). Viruses and cells were incubated for 72 hours before the transduction efficiency quantification. A control experiment was performed in the same conditions without viruses.

Sample 2 is the control experiment for the continuous flow acoustic conditions: only cells were injected in the channel of the acoustophoresis device and exposed to acoustic waves.

Sample 3 was obtained under continuous flow acoustic conditions with cells and viruses injected in the channel of the acoustophoresis device at inlets 122 and 121 respectively, as shown in figure 3A, and then exposed to 1W acoustic waves to increase their proximity.

Sample 4 was obtained under continuous flow acoustic conditions with cells and viruses injected in the channel of the acoustophoresis device at inlets 122 and 121 respectively, as shown in figure 3A, and then exposed to 2W acoustic waves to increase their proximity.

All samples were collected from the channel outlets, placed in 24 wells cell culture plates, and incubated in a 37°C, 5% CO2 incubator for 72 hours before the transduction efficiency quantification by flow cytometry.

Samples (1-4) were then collected and placed in cytometry tubes. The tubes were analyzed in a cytometer (FACS CantoII). After a first passage, propidium iodide (PI) was added: 10 µg/ml solution per sample prior to the cytometry analysis to measure the dead cells in each sample, to be excluded later in statistical quantification. Transduction efficiency was quantified using the gate for viable singlet cells that were positive for the green fluorescent (GFP).

### Results

**Table II: Experimental conditions and transduction efficiency results**

| Experiment | Conditions | Acoustic power | Total flow rate ml/min | MOI | Viable cells % | Transduction efficiency % |
|---|---|---|---|---|---|---|
| 1-1 | Static | - | - | 0.7 | 96.7 | 36.1 |
| 1-2 (control) | continuous flow | 1W | 1ml/min | 0.7 | 93.1 | 0.83 |
| 1-3 | continuous flow | 1W | 1ml/min | 0.7 | 88.2 | 61.20 |
| 1-4 | continuous flow | 2W | 1ml/min | 0.7 | 75.4 | 40.40 |
| 2-1 | Static | - | - | 0.7 | 90.8 | 36.3 |
| 2-2 (control) | continuous flow | 1W | 1ml/min | 0.7 | 95.7 | 0 |
| 2-3 | continuous flow | 1W | 1ml/min | 0.7 | 89.5 | 61.60 |
| 2-4 | continuous flow | 2W | 1ml/min | 0.7 | 97.3 | 50.03 |
| 3-1 | Static | - | - | 0.7 | 96.7 | 24. |
| 3-2 (control) | continuous flow | 1W | 1ml/min | 0.7 | 93.1 | 0.33 |
| 3-3 | continuous flow | 1W | 1ml/min | 0.7 | 88.2 | 72.50 |
| 3-4 | continuous flow | 2W | 1ml/min | 0.7 | 78.3 | 66.4 |

Table II shows the experimental conditions and the transduction efficiency results on samples obtained with static conditions or continuous flow acoustic conditions with an acoustic power from 0W to 2W, experiments were repeated thrice. Static conditions (noted "static" in Table II) refer to conditions wherein the cells and viruses were incubated in the cell culture dish, whereas continuous flow acoustic conditions (noted "continuous flow" in Table II) refer to conditions wherein cells and viruses were injected inside the channel of the acoustophoresis device and exposed to acoustic waves.

In each set of experiments (1-4), experimental conditions of Table I were reproduced, resulting in 4 samples per experiments: one sample from static conditions (-1), one control sample with continuous flow acoustic conditions (no virus, -2) and two samples from continuous flow acoustic conditions (-3 and -4).

In static experiments, i.e. performed in static conditions, cells and viruses were added in a 24 well cell culture plate with no acoustic exposure. In continuous flow acoustic, cells and viruses were injected into the inlets 122 as referenced in figure 3A. Control experiments (noted "(control)" in Table II) refer to experiments performed without virus, only cells.

In experiments (1-3, 1-4), (2-3, 2-4) and (3-3, 3-4), cells were injected with a 0.25 ml/min flow rate into each of the lateral inlets 122 as referenced in figure 3A. So, the total cell flow rate was 0.5 ml/min. Viruses conjugated with green fluorescent protein (GFP) were injected into the central inlet 121 at the same flow rate of 0.5 ml/min. This ensures that when the cell fluid layers and the virus fluid layer flow in the channel, there is no perturbation of the laminar flow, the fluid layers are parallel and stable. Both viruses and cells are exposed to acoustic waves with 1W/2W of electric power for about 30s. Frequency sweeping is performed as detailed hereabove. The force generated by acoustophoresis causes cells to move towards the center of the channel and be deflected to the virus layer, the cell/virus proximity thus increases, and transduction occurs. Samples were then collected from all outlets and the transduction efficiency was analyzed by flowcytometry. Since most of the viruses will be in outlet 131, sample collected at this outlet was used as the result of the acoustic experiments. However, suspensions collected at other outlets were analyzed. A fix amount of MOI= 0.7 has been used for all the 3 experiments using the same VLPs-VSV (GFP) viruses. The percentage of cell viability shows the percentage of the live/viable cells using the Propidium Iodide (PI) solution staining measured by flow cytometry in all samples. The percentages of transduction efficiency correspond to the viable cells that were positive for the GFP.

It is clear from Table II that continuous flow acoustic conditions with an acoustic power of 1W improves greatly the transduction efficiency and cell viability.

### Example 2: Comparative example between transduction with frequency sweeping and without frequency sweeping

### Materials and Methods

### Static conditions

Each test has been performed with a control run and static run, both without exposure to acoustic waves. For the control run, viruses were added to cells in a petri dish, then washed out immediately. For the static run, viruses were added to the cells inside a tissue culture Petri dish and then washed out after 90 mins. For both the control and static runs, a MOI of 25 has been used. For these tests, the baculovirus has been used for the transduction of green fluorescent protein (GFP) to the Jurkat T-cells.

**Table III below shows the transduction efficiency result of 4 repeated experiments.**

| Test | Run | Transduction efficiency (%) |
|---|---|---|
| 1 | control | 1.6 |
| 1 | Static | 17.5 |
| 2 | Control | 1.3 |
| 2 | Static | 18.3 |
| 3 | Control | 2.9 |
| 3 | Static | 14.9 |
| 4 | Control | 2.9 |

It is clear from Table III that allowing viruses and cells to be in a Petri dish for a longer time increases the transduction efficiency.

### Continuous flow conditions - with frequency sweeping (method of the invention)

Experiment 3 from example 1 was reproduced with Jurkat E6 cells in RPMI medium as cells suspension and lentiviral gene conjugated the green fluorescent protein (GFP) as virus suspension.

The acoustic channel of the acoustophoresis device is a molded PMMA (Poly(methyl methacrylate)) channel in two parts, bonded together with chloroform and epoxy glue. The geometry is identical to figure 3A. The distance between walls 111 and 112 is 750 µm and the width of the channel is 10 mm. The flow rate of virus suspension at inlet 121 is 0.5 ml/min, while the flow rate of cells suspension at each inlet 122 is 0.25 ml/min (flow rate for cells suspension thus being 0.5 ml/min), so the total flow rate of suspension entering the channel is 1 ml/min.

Acoustic waves frequency sweeping was performed, said frequency sweeping comprising 2 parts defining a sweeping cycle:
- a first part with a starting frequency f₁ of 0.9 MHz and an ending frequency f₂ of 1.1 MHz (see fig 5A and 5B for an illustration with different frequencies), using a sweep time of 1 ms;
- a second part called "return sweep", with a starting frequency f₂ of 1.1 MHz and an ending frequency f₁ of 0.9 MHz, using a sweep time of 1 ms;
- total sweep time for one sweeping cycle with part one and part two is then 2 ms;
- the signal is a continuous wave, i.e. the sweeping cycle is repeated continuously, around 500 times per second.

### Continuous flow conditions - without frequency sweeping

Jurkat E6 cells in RPMI medium and lentiviral gene conjugated the green fluorescent protein (GFP) were used as cells suspension virus suspension respectively.

An acoustic chamber including an ultrasonic transducer and a reflector was used. Viruses and cells were placed in the acoustic chamber in order to form a closed environment. A recirculation loop drew fluid from one end of the acoustic chamber and then circulated the fluid back through the other end into the acoustic chamber. The acoustic chamber had a volume of about 1 ml, and the recirculation loop had a volume of about 3 ml. Transduction test was performed using the acoustic chamber. Acoustic power was 1W.

### Results

**Table IV: Results from experiments performed in continuous flow conditions with or without frequency sweeping**

| | Without frequency sweeping | With frequency sweeping (method of the invention) |
|---|---|---|
| Cells/minute | 20 000-33 000 | 50 000-150 000 |
| MOI | 10 | 0.7-1 |
| Transduction efficiency | Times 2.0 increase (from 17.5% in the control to 35 % with acoustics) | Times 2.6 increase (from average 25.7% in the control to average 68% with acoustics, see fig 6) |

The experiment performed with frequency sweeping used 10 times less virus (MOI) for effective transduction compared to experiment performed without frequency sweeping. The method of the invention is thus suitable for therapeutical application specifically with CAR T-cells. The range of the cell concentration used in the experiment performed with frequency sweeping, resulting in a cell treatment speed of between 50 000 and 150 000 cells/min allows to scale up the experiment to handle higher number of cells and viral particles compared to experiment performed without frequency sweeping. The method of the invention treats 1.5 to 4.5 times more cells per minute, and the transduction gain is 30% higher in this case (2.6 gain against 2.0).

### Example 3:

### Materials and Methods

Experiment 3 from example 1 was reproduced with Jurkat E6 cells in RPMI medium as cells suspension and lentiviral gene conjugated the green fluorescent protein (GFP) as virus suspension.

The acoustic channel of the acoustophoresis device is a molded PMMA (Poly(methyl methacrylate)) channel in two parts, bonded together with chloroform and epoxy glue. The geometry is identical to figure 3A. The distance between walls 111 and 112 is 750 µm and the width of the channel is 10 mm. The flow rate of virus suspension at inlet 121 is 0.5 ml/min, while the flow rate of cells suspension at each inlet 122 is 0.25 ml/min (flow rate for cells suspension thus being 0.5 ml/min), so the total flow rate of suspension entering the channel is 1 ml/min.

Acoustic waves frequency sweeping was performed, said frequency sweeping comprising 2 parts defining a sweeping cycle:
- a first part with a starting frequency f₁ of 0.9 MHz and an ending frequency f₂ of 1.1 MHz (see fig 5A and 5B for an illustration with different frequencies), using a sweep time of 1 ms;
- a second part called "return sweep", with a starting frequency f₂ of 1.1 MHz and an ending frequency f₁ of 0.9 MHz, using a sweep time of 1 ms;
- total sweep time for one sweeping cycle with part one and part two is then 2 ms;
- the signal is a continuous wave, i.e. the sweeping cycle is repeated continuously, around 500 times per second.

### Results

All the 3 repeats of acoustic experiment gave a reproducible result in terms of transduction enhancement with 1W acoustic power.

Figure 6 represents the transduction efficiency results of all 3 experiments that are showed on table III in the experimental condition that is described in table II. The transduction efficiency percentage is a quantitative measurement of the transduction efficiency by flow cytometry showing the percentage of the live T-cells that were GFP positive, A: control sample cells without acoustic in static condition in the tissue culture well plates only cells and no virus, B: cells in static condition, no acoustic in the tissue culture well plates cells and the viral vector. Cells and viruses encounter increases the transduction efficiency 2.5 fold compared to the control status condition from an average efficiency of 35.5% to average efficiency of 68.3% when the acoustic power of 1W is applied to the acoustic channel showed in column E. An increase in acoustic power increases the transduction efficiency from 35.5% to 56.67% showed in column H. The expression of GFP was tested in cells collected from the outlet 132 to verify that there would be less virus in these outlets as shown in figure 6 columns D, F when 1W is applied and G and I when 2W is applied. The increase in transduction efficiency was not as high as the outlets 131, and there is less cell concentration in both outlets 132 compared to outlet 131. This proves that cells injected at the inlets 122 do in fact migrate to the center layer of the channel and mix more easily with the viruses than with no ultrasounds.

### REFERENCES

1 - Acoustophoresis device
11 - Chamber
111 - Transfer wall
112 - Opposite wall
121 - First inlet
122 - Second inlet
131 - First outlet
132 - Second outlet
14 - Acoustic wave generator
15 - Focusing plane
2 - Cell
3 - Foreign nucleic acid
A1 - Longitudinal axis
A2 - First transverse axis
A3 - Second transverse axis

## Claims

1. A method for introducing foreign nucleic acids (3) into cells (2) comprising:
i. providing an acoustophoresis device (1) comprising:
- a chamber (11) configured to be associated with at least one acoustic wave generator (14) for generating acoustic waves within the chamber (11);
- at least one acoustic wave generator (14) configured to generate acoustic waves within the chamber (11);
- at least two inlets (121, 122) in fluid communication with the chamber (11), said inlets (121, 122) comprising a first inlet (121) and at least one second inlet (122);
- at least two outlets (131, 132) in fluid communication with the chamber (11), said outlets (131, 132) comprising a first outlet (131) and at least one second outlet (132);
ii. injecting a suspension comprising foreign nucleic acids (3) in the chamber (11) at the first inlet (121) and injecting a cell (2) suspension in the chamber (11) at the at least one second inlet (122);
iii. applying an acoustic field by generating acoustic waves inside the chamber (11);
iv. sweeping the acoustic waves frequency from a first frequency f₁ to a second frequency f₂ using a sweep time ranging from 1 ms to 100 ms, f₂ being superior to f₁;
v. collecting a suspension comprising cells (2) modified by foreign nucleic acids (3) at the first outlet (131).

2. The method according to claim **1**, wherein the nucleic acids (3) are carried in a viral vector, or a microvesicle.

3. The method according to claim **2**, wherein multiplicity of infection is ranging from 0.5 to 30.

4. The method according to claim **1**, wherein the nucleic acids (3) are naked nucleic acids.

5. The method according to any one of claims **1** to **4**, wherein first frequency f₁ and second frequency f₂ are each ranging from 0.1 MHz to 4 MHz.

6. The method according to any one of claims **1** to **5**, wherein said method further comprises an additional step after step (iv) of sweeping the acoustic waves frequency from the second frequency f₂ to a third frequency f₃ using a sweep time ranging from 0.5 ms to 10 ms, f₂ being superior to f₃.

7. The method according to claim **6**, wherein first frequency f₁ is equal to third frequency f₃.

8. The method according to any one of claims **1** to **7**, wherein power applied to the acoustic wave generator (14) is ranging from 0.2 W to 50 W.

9. The method according to any one of claims **1** to **8**, wherein the cells (2) are selected among Chinese hamster ovary (CHO) cells, NSO hybridoma cells, baby hamster kidney (BHK) cells, human cells, regulatory T- cells, helper T-cells, cytotoxic T-cells, memory T-cells, effector T-cells, gamma delta T- cells, Jurkat T-cells, CAR-T cells, B cells, or NK cells, peripheral blood mononuclear cells (PBMCs), HEK293T, algae, plant cells, Tumor Infiltrating Lymphocytes (TIL), or bacteria.

10. A method for performing transduction of cells (2), comprising:
i. providing an acoustophoresis device (1) comprising:
- a chamber (11) configured to be associated with at least one acoustic wave generator (14) for generating acoustic waves within the chamber (11);
- at least one acoustic wave generator (14) configured to generate acoustic waves within the chamber (11);
- at least two inlets (121, 122) in fluid communication with the chamber (11), said inlets (121, 122) comprising a first inlet (121) and at least one second inlet (122);
- at least two outlets (131, 132) in fluid communication with the chamber (11), said outlets (131, 132) comprising a first outlet (131) and at least one second outlet (132);
ii. injecting a suspension of viral vectors comprising nucleic acids (3) in the chamber (11) at the first inlet (121) and injecting a cell (2) suspension in the chamber (11) at the at least one second inlet (122);
iii. applying an acoustic field by generating acoustic waves inside the chamber (11);
iv. sweeping the acoustic waves frequency from a first frequency f₁ to a second frequency f₂ using a sweep time ranging from 1 ms to 100 ms, f₂ being superior to f₁;
v. collecting a suspension comprising cells (2) modified by nucleic acids (3) at the first outlet (131).

11. The method according to claim **10**, wherein first frequency f₁ and second frequency f2 are each ranging from 0.1 MHz to 4 MHz.

12. The method according to claim **10** or claim **11**, wherein said method further comprises an additional step after step (iv) of sweeping the acoustic waves frequency from the second frequency f₂ to a third frequency f₃ using a sweep time ranging from 0.5 ms to 10 ms, f₂ being superior to f₃.

13. The method according to any one of claims **10** to **12**, wherein the cells (2) are selected among Chinese hamster ovary (CHO) cells, NSO hybridoma cells, baby hamster kidney (BHK) cells, human cells, regulatory T- cells, helper T-cells, cytotoxic T-cells, memory T-cells, effector T-cells, gamma delta T- cells, Jurkat T-cells, CAR-T cells, B cells, or NK cells, peripheral blood mononuclear cells (PBMCs), algae, plant cells, HEK293T, Tumor Infiltrating Lymphocytes (TIL), or bacteria.

14. Cells obtained by the method according to any one of claims **1** to **9**.

15. An acoustophoresis device (1) for introducing foreign nucleic acids (3) into cells (2) comprising:
- a chamber (11) configured to be associated with at least one acoustic wave generator (14) for generating acoustic waves within the chamber (11), said chamber (11) extending along a longitudinal axis;
- at least one acoustic wave generator (14) configured to generate acoustic waves within the chamber (11);
- at least two inlets (121, 122) in fluid communication with the chamber (11), said inlets (121, 122) comprising a first inlet (121) for introducing a suspension comprising foreign nucleic acids (3) in the chamber (11), and at least one second inlet (122) for introducing a cell (2) suspension in the chamber (11);
- at least two outlets (131, 132) in fluid communication with the chamber (11), said outlets (131, 132) comprising a first outlet (131) for collecting a suspension comprising cells (2) modified by foreign nucleic acids (3) and at least one second outlet (132);
wherein the at least one acoustic wave generator (14) is configured to sweep the acoustic waves frequency from a first frequency f₁ to a second frequency f2 using a sweep time ranging from 1 ms to 100 ms, f₂ being superior to f₁.
